# FASCICULE DE BREVET EUROPEEN

(11) **EP 2 723 250 B1**
(45) Date de publication et mention de la délivrance du brevet: **08.05.2019**
(21) Numéro de dépôt: 12738499.8
(22) Date de dépôt: 15.06.2012
(51) Int. Cl.: A61B 17/11, A61F 5/00, A61F 2/04

(54) **DISPOSITIF ANASTOMOTIQUE ET PROCEDE DE FABRICATION D'UN TEL DISPOSITIF**
ANASTOMOSEVORRICHTUNG UND HERSTELLUNGSVERFAHREN DAFÜR
ANASTOMOTIC DEVICE AND METHOD FOR MANUFACTURING SUCH A DEVICE

(30) Priorité: 22.06.2011 FR 1155508
(43) Date de publication de la demande: 30.04.2014
(73) Titulaire: Cousin Biotech, 59117 Wervicq Sud (FR); Université de Lille, 59800 Lille (FR)
(72) Inventeur: DALLE, Valery, 59170 Croix (FR); SOLECKI, Gilles, 59390 Lannoy (FR); PATTOU, François, 59000 Lille (FR); HIMPENS, Jacques, 9921 Vinderhoute (BE)
(74) Mandataire: Balesta, Pierre
(86) Numéro de dépôt international: PCT/FR2012/051347
(87) Numéro de publication internationale: WO 2012/175847

(56) Documents cités:
- WO-A1-98/19632
- WO-A1-2009/012250
- US-A- 5 976 178
- US-A1- 2010 318 015

## Description

La présente invention concerne le domaine technique des dispositifs médicaux anastomotiques pour joindre des viscères creux, aptes à être déployé à la surface de deux lumens adjacents superposés.

L'anastomose chirurgicale est un abouchement chirurgical de deux conduits, réalisé par suture manuelle ou par agrafage. La suture est effectuée avec du fil qualifié de résorbable, car il disparaît de lui-même avec le temps à l'intérieur de l'organisme. Ces sutures sont utilisées en chirurgie gastro-entérologique (spécialité médicale du tube digestif) et en chirurgie urologique en ce qui concerne les voies urinaires. Quelquefois les anastomoses chirurgicales se font avec du fil non résorbable, en particulier s'agissant de l'anastomose de vaisseaux, notamment des artères.

L'anastomose dans le domaine viscéral est utilisée pour rétablir la continuité du tube digestif après que l'on ait procédé à une ablation d'une partie ou de la totalité d'un organe creux, comme par exemple l'estomac, l'intestin grêle ou le côlon.

L'anastomose dans le domaine viscéral est également utilisée pour contourner un obstacle sur la voie digestive. Si le chirurgien découvre un obstacle sur la voie digestive, comme par exemple une tumeur de la tête du pancréas venant boucher le duodénum, il va pratiquer alors une gastroentérostomie qui va permettre de relier l'estomac à l'intestin grêle. Quelquefois l'obstacle se situe sur les voies biliaires et nécessite alors une dérivation à l'intérieur du tube digestif. Le chirurgien pratique alors une anastomose biliodigestive.

Les différents types d'anastomoses viscérales sont les suivantes :
- L'anastomose oeso-gastrique se fait entre l'oesophage et l'estomac,
- L'anastomose gastro-jéjunale se fait entre l'estomac et le jéjunum,
- L'anastomose duodeno-jéjunale se fait entre le duodénum et le jéjunum,
- L'anastomose iléocolite se fait entre l'iléon et le côlon,
- L'anastomose colo-colique se fait entre deux parties du côlon,
- L'anastomose iléo-rectale se fait entre l'iléon et le rectum.

Dans le cadre de la présente invention, le dispositif anastomotique désigne le dispositif médical qui permet de créer de façon artificielle une anastomose entre deux conduits anatomiques sans utilisation d'agrafes ou de fils de sutures. De tels dispositifs anastomotiques peuvent être utilisés par exemple dans le traitement de l'obésité, en particulier dans le traitement de l'obésité morbide pour la mise en oeuvre de la technique dite du bypass gastrique de type « Roux en Y ». Rappelons que le pourcentage de la population mondiale souffrant d'obésité morbide est en constante augmentation. Il est ainsi estimé que le nombre de personnes souffrant d'obésité morbide rien qu'aux Etats-Unis dépassent 10 millions. Les personnes souffrant d'obésité morbide présentent un risque accru de développer du diabète, des maladies respiratoires et cardiaques, des accidents cérébrovasculaires, de l'hypertension, de l'arthrose, des apnées du sommeil ou encore certaines formes de cancer (de l'utérus, du sein, de la vésicule biliaire, du rein, du colon ...). La technique dite du bypass gastrique de type « Roux en Y » peut être pratiquée par coelioscopie et consiste à réduire le volume de l'estomac et à modifier le circuit alimentaire. Ce type de bypass agit par plusieurs mécanismes qui associent une restriction de l'estomac et un retard de l'assimilation des aliments par l'organisme humain.

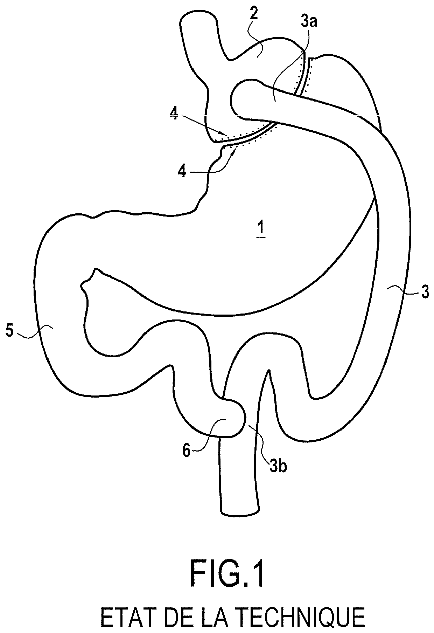

Tel que cela est représenté à la figure 1 annexée à la présente, cette technique du bypass « Roux en Y» consiste à pratiquer une section transversale de l'estomac 1 afin de créer une poche proximale gastrique 2 au minimum d'environ 15 ml dans laquelle arrivent les aliments, reliée directement au jéjunum 3 (partie moyenne du petit intestin grêle). Les aliments ne passent plus par l'estomac 1. Les zones 4 de l'estomac 1 sectionnées sont généralement refermées à l'aide d'agrafes. Les anastomoses gastro-jéjunales et duodeno-jéjunales sont realisées très souvent par le biais de sutures. De ce fait, ces anastomoses sont souvent sujettes à des fuites pouvant être à l'origine de péritonites, d'ulcères, ou de rétrécissements. Le retard de l'assimilation des aliments par l'organisme n'est que la conséquence d'un retard de contact du bol alimentaire avec les sécrétions biliaires et pancréatiques qui se déversent dans le duodénum 5. Dans un bypass de type « Roux en Y », ce contact du bol alimentaire avec les sécrétions biliaires et pancréatiques ne se fait qu'à la jonction de l'anastomose duodeno-jejunale. La malabsorption des aliments est également la conséquence d'une diminution de la longueur du jéjunum dans lequel se déverse normalement les sécrétions biliaires et pancréatiques. En effet, une partie de jéjunum 3 d'une longueur comprise entre 1 et 1,5 mètres est anastomosée au niveau de la poche proximale gastrique 2. En fonction de la grandeur de la poche gastrique 2 créée, le caractère restrictif de l'opération est plus ou moins prononcé. Le court-circuit d'une partie de l'intestin grêle entraîne donc une diminution de la réabsorption des aliments et surtout des graisses.

Les portions supérieure 3a et inférieure 3b du jéjunum 3 doivent être raccordées respectivement à la poche gastrique 2 et à l'anse duodénale 6 de façon étanche afin d'éviter toute fuite du contenu digestif présentant notamment les inconvénients décrits ci-dessus. Un tel raccordement peut être effectué avec un dispositif anastomotique comprenant un corps tubulaire principal d'axe longitudinal (L), ayant des première et seconde extrémités ouvertes ainsi que des faces extérieure et intérieure opposées. Le corps tubulaire est disposé à travers les ouvertures ou lumens de deux parois anatomiques adjacentes, telles que la paroi de la poche gastrique 2 et la paroi de la portion 3a du jéjunum, d'une part, et, la paroi de la portion 3b du jéjunum 3 et la paroi de l'anse duodénale 6, d'autre part.

Le document WO 03/000142 décrit un dispositif anastomotique comprenant un corps tubulaire formé dans un tube tissé à partir d'un alliage à mémoire de forme. Cet alliage dit à mémoire de forme est composé de titane et de nickel avec une température de fin de transition austénite supérieure à la température du corps humain. De ce fait, ce dispositif présente une première forme tubulaire à une température inférieure à celle du corps humain. Lorsqu'il est sorti de son introducteur, puis soumis ensuite à une température proche de celle du corps humain, le tube adopte une seconde forme différente de la première forme tubulaire, ledit tube se contracte longitudinalement permettant de déployer transversalement des pétales se projetant de façon perpendiculaire à l'axe longitudinal du tube. Les pétales viennent ainsi en appui de part et d'autre de la périphérie des deux lumens en apposition. Ce dispositif ne peut pas être posé facilement dans sa forme d'implantation, laquelle correspond à ladite seconde forme, s'il est fabriqué dans un alliage titane nickel superélastique. En effet, un alliage superélastique est composé de titane et de nickel et a la particularité de posséder une température de fin de transition austénite très inférieure à la température du corps. Dès la sortie de son introducteur, qui a permis le cheminement dans l'organisme, les pétales se déploieraient transversalement avant même que les parties proximale et distale du dispositif anastomotique ne soient complètement sorties de l'introducteur. Pour obtenir un déploiement correct, il est important que le dispositif sorte intégralement de l'introducteur dans sa première forme tubulaire avant que les pétales ne se forment en commençant leur déploiement radial par les extrémités distale et proximale du dispositif. Pour cette raison, la forme décrite dans le brevet WO 03/000142 ne peux fonctionner que sous la condition d'être réalisée en alliage à mémoire de forme. Il existe donc un risque que le dispositif anastomotique ne se déploie pas correctement et ainsi ne se positionne pas correctement à la surface des deux lumens en apposition à raccorder.

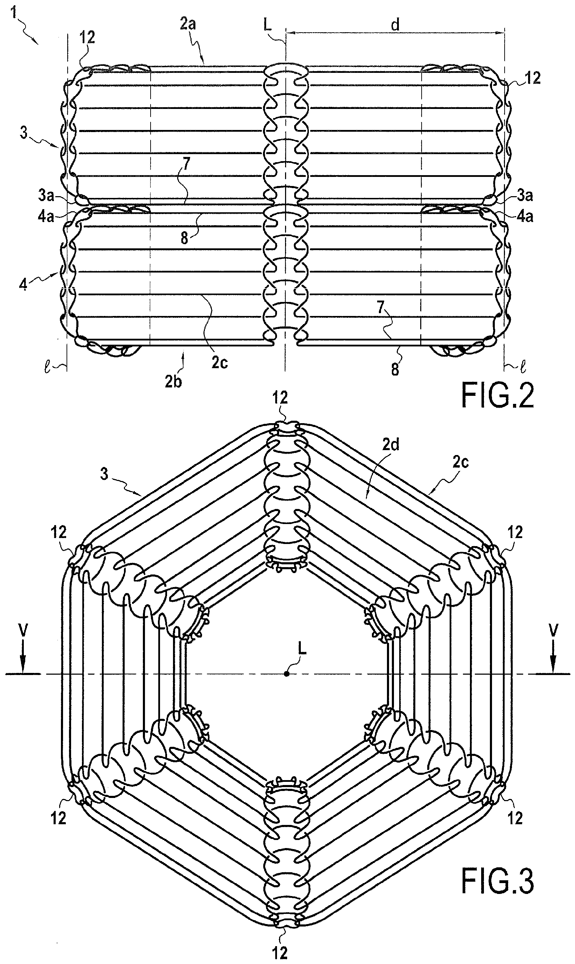

Ce dispositif anastomotique présente en outre l'inconvénient majeur d'exercer des pressions ponctuelles très importantes à l'extrémité des pétales en forme de pointe (voir FIG 3B partie 20'). Ces pressions importantes ponctuelles sont à l'origine de nécroses des tissus. Les tissus placés entre une première série de pétales en forme de pointe d'un premier groupe opposé à des pétales d'un second groupe peuvent laisser place à des nécroses de forme circulaire. Il faut donc un dispositif anastomotique qui soit suffisamment compressif pour réaliser la cicatrisation des tissus mais pas trop pour éviter leur nécroses.

Le document WO 2009/012250 décrit un rivet destiné à joindre deux lumens ou viscères creux et le système d'introduction dudit rivet présentant les mêmes inconvénients que le document WO 03/000142 précité. Le rivet a une première forme tubulaire ayant des extrémités ouvertes telles que représentées à la figure 1A dans un état non déployé. Dans son état déployé, le rivet forme deux zones d'expansion 112A et 114A représentées par exemple à la figure 3E par le déploiement de pétales grâce à des fentes 104 et 106 ménagées dans le rivet 100. Le déploiement des pétales est obtenu par la mise en oeuvre du système d'introduction 200 comprenant un guide d'introduction 202 pourvues d'au moins deux joues 254 permettant lors de l'introduction du rivet sur le site d'implantation de le déformer localement au niveau des fentes et ainsi de créer des zones d'expansion.

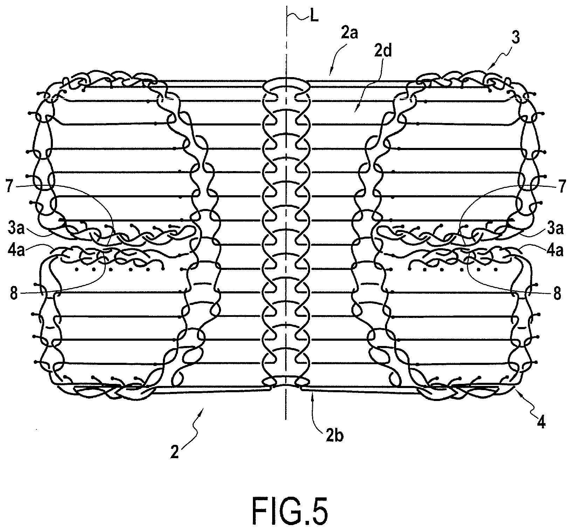

Dans une nième variante représentée aux figures 5A à 5D, le rivet peut être une colonne cylindrique dans une grille ou selon un autre arrangement de câble similaire à un stent ouvert aux extrémités. Il est possible de s'affranchir du guide d'introduction par l'utilisation d'un matériau à mémoire de forme, tel que le nitinol (ce qui correspond à un alliage titane nickel superélastique). Le rivet est ainsi introduit selon une forme générale tubulaire puis développe, une fois sur le site d'implantation, deux zones d'expansion 504 et 506.

Le document WO 00/27313 décrit également un dispositif anastomotique dans le domaine vasculaire comprenant un tube dans un alliage plastique qui permet un déploiement à l'aide d'un ballonnet d'expansion placé à l'intérieur du dispositif et retiré après l'expansion du dispositif. Ce dispositif, déformable plastiquement, ne possède pas de force axiale suffisante exercée selon son axe longitudinal après déploiement pour permettre le rapprochement de lumens en apposition. Pour cette raison, ce dispositif possède des ancres en partie pointues (voir FIG 10 référence 42) permettant une connexion plus efficace des deux lumens Ce dispositif a pour inconvénient que les ancres pointues ne peuvent pas être employées dans les réalisations d'anastomoses viscérales sans avoir des déchirements importants de la paroi entérique. Les mouvements péristaltiques des viscères sont en effet très importants et d'une grande amplitude. Il n'est ainsi pas envisageable d'utiliser des ancres pointues sans engendrer des perforations importantes ou le déchirement des viscères.

La présente invention a pour objet de proposer un dispositif anastomotique limitant les risques de nécroses des surfaces des lumens à maintenir ensemble, pouvant être déployé de façon fiable d'un introducteur quel que soit le type de matériau(x) entrant dans sa fabrication (mémoire de forme ou superélastique).

La présente invention a également pour objet un dispositif anastomotique pouvant être fabriqué à la fois dans un alliage à mémoire de forme ou un matériau super élastique.

La présente invention a ainsi pour objet un dispositif anastomotique comprenant un corps tubulaire principal d'axe longitudinal (L), ayant des première et seconde extrémités ouvertes ainsi que des faces extérieure et intérieure opposées. Avantageusement, lesdites première et seconde extrémités se prolongent par des premier et second rebords annulaires se projetant de la surface extérieure dudit corps tubulaire principal, ayant une configuration en U retourné en sorte de former des première et seconde zones annulaires d'appui. Ledit corps principal et lesdits premier et second rebords sont formés à partir d'une structure tricotée de forme tubulaire comprenant au moins deux colonnes de mailles (12), d'axe longitudinal (l) parallèle audit axe (L) en sorte que ledit dispositif (1) puisse s'allonger selon ledit axe longitudinal (L) entrainant la diminution du diamètre au repos (r) du corps tubulaire principal (2) sous l'application d'une tension longitudinale exercée manuellement par le praticien.

Cette tension longitudinale est ainsi de préférence inférieure ou égale à 20 daN, encore de préférence inférieure ou égale à 10 daN, et encore de préférence inférieure ou égale à 2 daN.

Avantageusement, lorsqu'une tension longitudinale, par exemple une simple tension manuelle, est exercée selon l'axe longitudinal (L) sur le corps tubulaire principal ou les rebords annulaires, les mailles disposées selon lesdites colonnes de mailles glissent les unes par rapport aux autres, en sorte que le dispositif augmente sa hauteur (H) au repos entraînant également une diminution de son diamètre au repos (r0). Cette disposition permet de minimiser l'encombrement du dispositif anastomotique selon l'invention et facilite ainsi son introduction dans des trocarts de faibles diamètres.

La combinaison de la structure tricotée du corps tubulaire principal et d'au moins deux colonnes de mailles disposées selon l'axe longitudinale (L), permet de diminuer le diamètre au repos du dispositif lorsqu'une traction est exercée sur le corps tubulaire facilitant son introduction dans un trocart sans pour autant défaire les rebords annulaires et modifier les plans d'appui. Le dispositif selon l'invention récupère son diamètre initial au repos lors de sa mise en place pour le rapprochement des deux viscères creux à joindre.

Le rivet décrit dans WO 2009/012250 , en particulier en référence à la figure 5A, n'est pas dans une structure tricotée de forme tubulaire et ne présente pas au moins deux colonnes de mailles le long desquelles les fils glissent entraînant ainsi une diminution du diamètre au repos facilitant l'introduction du dispositif et permettant de fabriquer un dispositif dans n'importe quel matériau.

En outre, les premier et second rebords annulaires ayant une configuration en U retourné sont dans le prolongement et selon la circonférence des première et seconde extrémités ouvertes, respectivement. Les premier et second rebords annulaires viennent en appui selon des première et seconde zones d'appui de part et d'autre des lumens à maintenir en apposition, et sont ainsi au regard l'une de l'autre en maintenant ensemble les parois anatomiques desdits lumens.

Avantageusement, les premier et second rebords annulaires, de par leur forme en U retourné, en combinaison avec le fait qu'ils soient tricotés, exercent une pression sensiblement homogène selon les première et seconde zones annulaires d'appui sur les parois anatomiques bordant les lumens à maintenir en apposition, limitant ainsi les risques de nécrose.

Les premier et second rebords annulaires présentent une hauteur déterminée (h), de préférence sensiblement du même ordre et supérieure ou égale à 2 mm. Le corps tubulaire principal présente une hauteur déterminée (H), de préférence supérieure ou égale à 5 mm. La hauteur de l'intervalle dans lequel sont maintenues les parois anatomiques bordant les lumens en apposition correspond à la différence entre la hauteur (H) du corps tubulaire principal et la somme des hauteurs (h) des premier et second rebords annulaires.

La forme particulière du dispositif anastomotique et le fait qu'il soit formé à partir d'une structure tubulaire tricotée font qu'il peut être déployé facilement d'un introducteur quel que soit le type de fil employé (matériau super élastique ou à mémoire de forme) pour fabriquer la structure tubulaire tricotée.

Les première et seconde extrémités sont ouvertes en sorte d'être en communication fluidique.

Dans une variante, les première et seconde extrémités libres des premier et second rebords se prolongent respectivement par des première et seconde branches convergeant vers la surface extérieure dudit corps tubulaire principal, de préférence lesdites première et seconde branches sont dans des plans (P1,P2) sensiblement perpendiculaire à l'axe longitudinal (L) dudit corps tubulaire principal.

Les première et seconde branches prolongent les première et seconde annulaires d'appui en sorte de former des première et seconde surfaces d'appui. Lesdites surfaces d'appui exercent des pressions selon les parois anatomiques à maintenir rapprochées, ce qui améliore la répartition des pressions de maintien exercées et limite les risques de nécrose.

Les parois anatomiques bordant les lumens en apposition sont ainsi maintenues dans l'intervalle de hauteur séparant lesdites première et seconde surfaces d'appui, au regard l'une de l'autre.

De préférence, les première et seconde branches sont formées à partir de la même structure tricotée de forme tubulaire que celle utilisée pour le corps principal tubulaire et les premier et second rebords annulaires.

Dans une variante, le corps principal, les premier et second rebords, éventuellement les première et seconde branches, comprennent des colonnes de mailles espacées d'une distance (d) supérieure ou égale à 2 mm, de préférence supérieure ou égale à 5 mm.

Dans une variante, le corps principal, les premier et second rebords, éventuellement les première et seconde branches, comprennent au moins six colonnes de mailles.

Cette disposition permet de répartir au mieux les pressions exercées sur les parois anatomiques bordant les lumens en apposition.

Dans une variante, lesdites première et seconde branches ont leurs extrémités libres enroulées sur elles-mêmes afin d'ajuster les forces compressives sur les parois anastomotiques bordant les lumens en apposition.

Dans une variante, ladite au moins une structure tricotée de forme tubulaire comprend un ou des fil(s) monofilament(s) et/ou un ou des fil(s) multifilamentaire(s) dans un alliage super élastique.

L'alliage super élastique est choisi de préférence seul ou en combinaison parmi les alliages suivants : alliage Titane/Nickel à mémoire de forme ou inox ASI 316LVM .

Dans une variante, ladite au moins une structure tricotée de forme tubulaire comprend un ou des fil(s) monofilament(s) et/ou un ou des fil(s) multifilamentaire(s) dans un alliage à mémoire de forme.

L'alliage à mémoire de forme thermique est de préférence choisi seul ou en combinaison parmi les alliages suivants : alliage Titane/Nickel superélastique ou inox ASI 316LVM .

La présente invention a pour objet, selon un deuxième aspect, un dispositif implantable comprenant un dispositif anastomotique selon l'une des variantes de réalisation décrites ci-dessus, et une gaine tubulaire souple, de préférence dans un matériau textile, ayant des extrémités distale et proximale ouvertes, ladite extrémité proximale étant solidarisée ou apte à être solidarisée à ladite première extrémité du corps tubulaire principale.

Ladite gaine tubulaire souple peut être fabriquée à partir d'un panneau textile tricoté, tissé ou encore dans un nontissé dont les bords longitudinaux sont joints pour confectionner un tube. La gaine tubulaire souple peut également être fabriquée à partir d'une tresse, de silicone, ou d'une tresse revêtue de silicone. Cette gaine a pour fonction d'être implantée dans l'estomac et d'assurer la communication fluidique entre la sortie de l'oesophage débouchant normalement dans l'estomac et le dispositif anastomotique selon l'invention.

L'extrémité proximale de la gaine tubulaire peut directement être suturée au niveau de l'oesophage.

Dans une sous-variante, le dispositif implantable comprend une bague de maintien solidarisée ou apte à être solidarisée à ladite extrémité proximale de la gaine tubulaire, et configurée en sorte de se maintenir en appui contre les parois de l'oesophage.

Ladite bague permet de maintenir l'extrémité proximale de la gaine au niveau de l'oesophage. La gaine peut le cas échéant se prolonger dans les viscères après avoir traversé le dispositif anastomotique.

La présente invention a pour objet, selon un troisième aspect, un procédé de fabrication d'un dispositif anastomotique tel que décrit ci-dessus comprenant une première étape de fabrication d'au moins une structure tricotée de forme tubulaire d'axe longitudinal (L) ayant des première et seconde extrémités libres ouvertes ainsi que des faces extérieure et intérieure opposées, à partir de fil(s) monofilament(s) et/ou de fil(s) multifilamentaire(s). Avantageusement, ledit procédé comprend une seconde étape au cours de laquelle lesdites première et seconde extrémités libres sont enroulées sur elle-même, de préférence à l'aide d'une tige de mise en forme, puis thermofixées, en sorte de former respectivement des premier et second rebords annulaires se projetant de la face extérieure d'un corps tubulaire principal tricoté, lesdits premier et second rebords ayant une configuration en U retourné en sorte de former des première et seconde zones annulaires d'appui.

La température et la durée de thermofixation sont fonction du ou des matériaux composant le ou les fils entrant dans la structure tricotée de forme tubulaire. De préférence, la température de thermofixation est supérieure ou égale à 120°C. De préférence, le temps de la thermofixation est supérieur ou égal à 5 minutes.

Dans une variante, au cours de la seconde étape, lesdites première et seconde extrémités sont enroulées sur elles-mêmes en sorte que leurs bords libres convergent vers la face extérieure dudit corps tubulaire principal et forment respectivement des première et seconde branches convergeant vers la face extérieure dudit corps principal, de préférence lesdites première et seconde branches sont dans des plans (P1,P2) sensiblement perpendiculaires à l'axe longitudinal (L) dudit corps principal.

Dans une variante, le schéma de maille de la structure tricotée de forme tubulaire est un jersey avant ou arrière et est déterminé en sorte de ménager des colonnes de mailles distinctes et espacées d'une distance (d) supérieure ou égale à 2 mm, de préférence supérieure ou égale à 5 mm.

Ce schéma de mailles est préféré pour faciliter le glissement des mailles disposées selon des colonnes de mailles les unes par rapport aux autres et ainsi diminuer le diamètre au repos (r0) du corps tubulaire principal.

La présente invention sera mieux comprise à la lecture d'un exemple de réalisation, cité à but non limitatif et illustré dans les figures ci-après, annexées à la présente :
- La figure 1 représente de façon schématique un circuit gastrique de l'état de la technique formé après une intervention chirurgicale du type by-pass gastrique type « Roux en Y »;
- La figure 2 représente, de façon schématique et vu de côté, un premier exemple de dispositif anastomotique selon l'invention ;
- La figure 3 représente, de façon schématique et vu de dessus, le dispositif représenté à la figure 2 ;
- Les figures 4A et 4B représentent de façon schématique, respectivement vue de dessus puis de côté, le dispositif anastomotique représenté aux figures 2 et 3 ;
- La figure 5 représente de façon schématique et selon le plan de coupe V-V, le dispositif anastomotique représenté aux figures 2 à 4 ;
- La figure 6 représente un exemple de dispositif implantable selon l'invention, dans un exemple de circuit gastrique du type by-pass, comprenant le dispositif anastomotique représenté aux figures 1 à 5.

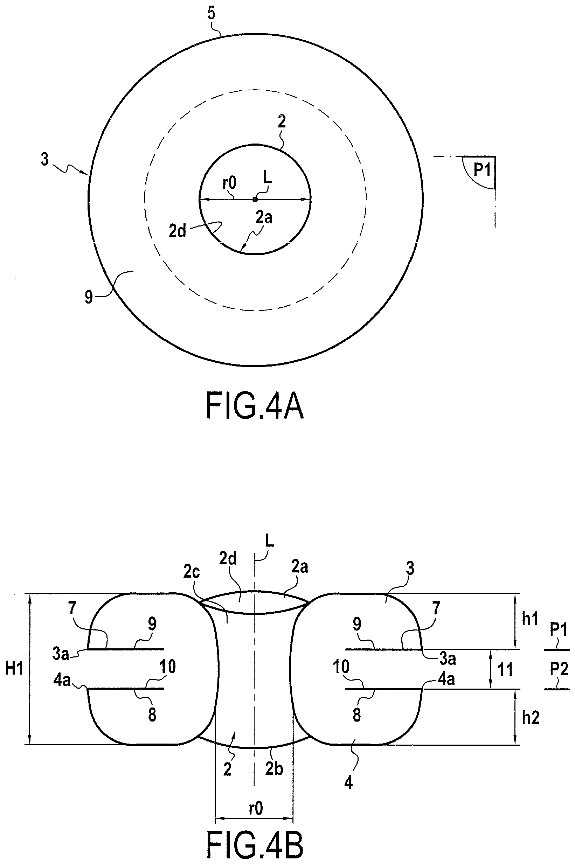

Le dispositif anastomotique 1 représenté à la figure 2 comprend un corps tubulaire principal 2 d'axe longitudinal (L), ayant des première 2a et seconde 2b extrémités ouvertes ainsi que des faces extérieure 2c et intérieure 2d opposées. Lesdites première 2a et seconde 2b extrémités se prolongent par des premier 3 et second 4 rebords annulaires se projetant de la face extérieure 2c dudit corps tubulaire principal 2, et ayant une configuration en U retourné telle que cela est visible aux figures 4B et 5. Les premier 3 et second 4 rebords annulaires forment des première et seconde zones annulaires d'appui, seule la première zone annulaire d'appui 5 a été représentée à la figure 4. Les première et seconde extrémités libres 3a, 4a respectivement des premier 3 et second 4 rebords se prolongent par des première 7 et seconde 8 branches convergeant vers la face extérieure 2c dudit corps tubulaire principal 2. Dans cet exemple précis, lesdites première 7 et seconde 8 branches sont respectivement dans des plans (P1,P2) sensiblement perpendiculaires à l'axe longitudinal (L) dudit corps tubulaire principal 2. Les première et seconde branches 7 et 8 forment respectivement des première 9 et seconde 10 surface d'appui. L'intervalle de hauteur 11 dans lequel les parois anatomiques bordant les lumens en apposition sont destinées à être maintenues, correspond à la hauteur H1 du corps tubulaire 2 moins la somme des hauteurs h1 et h2 respectivement des premier et second rebords annulaires 3 et 4.

Avantageusement, le corps principal 2, lesdits premier 3 et second 4 rebords ainsi que les branches 7 et 8 sont formés à partir d'une structure tricotée de forme tubulaire. Ainsi, le corps principal creux 2 et les premier 3 et second 4 rebords comprennent au moins deux colonnes de mailles. Dans cet exemple précis, six colonnes de mailles 12, d'axe longitudinale (l) parallèle audit axe (L) en sorte que ledit dispositif 1 puisse s'allonger selon ledit axe longitudinal (L) entrainant la diminution du diamètre au repos (r0) du corps tubulaire principal 2 sous l'application d'une tension longitudinale (daN) exercée manuellement par le praticien. Les colonnes de mailles 12 sont espacées d'une distance (d) supérieure ou égale à 2 mm, de préférence supérieure ou égale à 5 mm.

La structure tricotée de forme tubulaire comprend un ou des fil(s) monofilament(s) et/ou un ou des fil(s) multifilamentaire(s) dans un alliage super élastique ou à mémoire de forme thermique, de préférence dans un alliage super élastique. On distingue bien ainsi, agencées selon les colonnes de mailles 12, les mailles imbriquées les unes dans les autres. La structure tricotée de forme tubulaire a pour schéma de mailles un jersey avant.

Lorsqu'une tension selon l'axe longitudinal (L) est appliquée manuellement par le praticien, cette dernière est transmise à l'axe longitudinal (l) des colonnes de mailles 12, le pied d'une maille glissera ainsi dans la tête de la maille qui lui est adjacente en sorte d'obtenir une augmentation de la hauteur H1 et ainsi la diminution de r0 facilitant alors l'insertion du dispositif anastomotique 1 dans un introducteur.

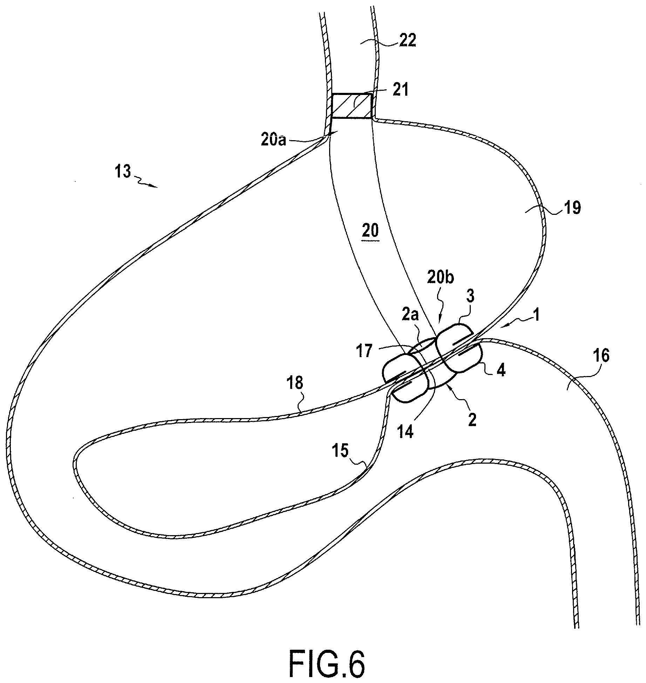

La figure 6 représente un dispositif implantable selon l'invention 13 comprenant le dispositif anastomotique 1 implanté en sorte de raccorder de façon étanche le lumen 14 dans la paroi 15 d'une portion 16 de l'intestin grêle et le lumen 17 dans la paroi 18 de l'estomac 19. Le dispositif implantable 13 comprend une gaine tubulaire souple 20, de préférence dans un matériau textile, ayant des extrémités proximale 20a et distale 20b. L'extrémité distale 20b est solidarisée ou apte à être solidarisée à la première extrémité 2a du corps tubulaire principale 2.

Le dispositif implantable 1 comprend également une bague de maintien 21 solidarisée ou apte à être solidarisée à ladite extrémité proximale 20a de la gaine tubulaire 20, et configurée en sorte de se maintenir en appui contre les parois de l'oesophage 22. La figure 6 représente une méthode de by-pass gastrique réduisant la prise alimentaire puisque la taille de l'estomac 19 est réduite au volume de la gaine souple 20 ainsi que l'absorption des aliments ingérés puisque la longueur d'intestin grêle dans laquelle les aliments passent est réduite à la portion 16. Cette technique a pour avantage qu'il n'est pas nécessaire de suturer, notamment à l'aide d'agrafes, l'estomac 19, en sorte de former une poche gastrique et ce de façon irréversible tel que cela est illustré à la figure 1. Il n'y pas à proprement parler de transsection de l'estomac 19. Une ouverture du lumen 17 est pratiquée dans la paroi 18 de l'estomac 19 en sorte de la faire correspondre avec une ouverture ou lumen 14 pratiquée dans la paroi 15 de la portion 16 de l'intestin grêle. La portion 16 de l'intestin grêle correspond également au jéjunum.

En fonctionnement, le dispositif anastomotique 1 maintient les parois 15 et 18 bordant les lumens 14 et 17 en apposition en sorte que la portion 16 de l'intestin grêle soit en liaison fluidique avec la gaine souple 20. Les premier 3 et second 4 rebords annulaires exercent des pressions opposées sur les parois anatomiques 15 et 18 via les première et seconde surfaces d'appui 9 et 10 dans le prolongement des zones annulaires d'appui 5 et 6, respectivement. Le dispositif anastomotique 1 étant formé à partir d'une structure tricotée de forme tubulaire, les pressions exercées par les surfaces d'appui 9 et 10 sont homogènes. De plus, le dispositif anastomotique 1 présente une bonne souplesse selon son axe longitudinale (L) lui permettant de résister aux compressions longitudinales (L) engendrées par les mouvements péristaltiques.

## Revendications

1. Dispositif anastomotique (1) pour joindre des lumens ou des viscères creux, apte à être déployé à la surface de deux lumens adjacents superposés, ledit dispositif (1) comprend un corps tubulaire principal (2) d'axe longitudinal (L), ayant des première (2a) et seconde extrémités (2b) ouvertes ainsi que des faces extérieure (2c) et intérieure (2d) opposées, dans lequel lesdites première (2a) et seconde (2b) extrémités se prolongent par des premier (3) et second (4) rebords annulaires se projetant de la face extérieure (2c) dudit corps tubulaire principal (2), ayant une configuration en U retourné en sorte de former des première (5) et seconde zones annulaires d'appui, **caractérisé en ce que** ledit corps principal (2) et lesdits premier (3) et second (4) rebords sont formés à partir d'une structure tricotée de forme tubulaire comprenant au moins deux colonnes de mailles (12), d'axe longitudinale (l) parallèle audit axe (L) en sorte que ledit dispositif (1) puisse s'allonger selon ledit axe longitudinal (L) entrainant la diminution du diamètre au repos (r) du corps tubulaire principal (2) sous l'application d'une tension longitudinale exercée manuellement par le praticien.

2. Dispositif anastomotique (1) selon la revendication 1, **caractérisé en ce que** les première (3a) et seconde (4a) extrémités libres des premier (3) et second (4) rebords se prolongent respectivement par des première (7) et seconde (8) branches convergent vers la surface extérieure (2c) dudit corps tubulaire principal (2), de préférence lesdites première (7) et seconde (8) branches sont dans des plans (P1,P2) sensiblement perpendiculaires à l'axe longitudinale (L) dudit corps tubulaire principal (2).

3. Dispositif anastomotique (1) selon l'une ou l'autre des revendications 1 et 2, **caractérisé en ce que** le corps principal (2), les premier (3) et second (4) rebords, éventuellement les première (7) et seconde (8) branches, comprennent des colonnes de mailles (12) espacées d'une distance (d) supérieure ou égale à 2 mm, de préférence supérieure ou égale à 5 mm.

4. Dispositif anastomotique (1) selon l'une des revendications 1 à 3, caractérisé en ce le corps principal (2), les premier (3) et second (4) rebords, éventuellement les première (7) et seconde (8) branches, comprennent au moins six colonnes de mailles (12).

5. Dispositif anastomotique (1) selon l'une des revendications 2 à 4, **caractérisé en ce que** lesdites première et seconde branches ont leurs extrémités libres enroulées sur elles-mêmes.

6. Dispositif anastomotique (1) selon l'une des revendications 1 à 5, **caractérisé en ce que** ladite au moins une structure tricotée de forme tubulaire comprend un ou des fil(s) monofilament(s) et/ou un ou des fil(s) multifilamentaire(s) dans un alliage super élastique.

7. Dispositif anastomotique (1) selon l'une ou l'autre des revendications 1 à 5, **caractérisé en ce que** ladite au moins une structure tricotée de forme tubulaire comprend un ou des fil(s) monofilament(s) et/ou un ou des fil(s) multifilamentaire(s) dans un alliage à mémoire de forme.

8. Dispositif implantable (1) comprenant un dispositif anastomotique selon la revendication 1, et une gaine tubulaire souple (20), de préférence dans un matériau textile, ayant des extrémités distale (20b) et proximale (20a) ouvertes, ladite extrémité distale (20b) étant solidarisée ou apte à être solidarisée à ladite première extrémité du corps tubulaire principal (2).

9. Dispositif implantable (1) selon la revendication 8, **caractérisé en ce qu'**il comprend une bague de maintien (21) solidarisée ou apte à être solidarisée à ladite extrémité proximale (20a) de la gaine tubulaire (20), et configurée en sorte de se maintenir en appui contre les parois de l'oesophage (22).

10. Dispositif implantable selon l'une ou l'autre des revendications 8 et 9, **caractérisé en ce qu'**il comprend un dispositif anastomotique selon l'une quelconque des revendications 2 à 7.

11. Procédé de fabrication d'un dispositif anastomotique (1) selon l'une quelconque des revendications 1 à 10, pour joindre des lumens ou des viscères creux, le dispositif étant apte à être déployé à la surface de deux lumens adjacents superposés, ledit dispositif (1) comprend un corps tubulaire principal (2) d'axe longitudinal (L), ayant des première (2a) et seconde extrémités (2b) ouvertes ainsi que des faces extérieure (2c) et intérieure (2d) opposées, lesdites première (2a) et seconde (2b) extrémités se prolongeant par des premier (3) et second (4) rebords annulaires se projetant de la face extérieure (2c) dudit corps tubulaire principal (2), ayant une configuration en U retourné en sorte de former des première (5) et seconde zones annulaires d'appui, **caractérisé en ce que** ledit corps principal (2) et lesdits premier (3) et second (4) rebords sont formés à partir d'une structure tricotée de forme tubulaire comprenant au moins deux colonnes de mailles (12), d'axe longitudinale (l) parallèle audit axe (L) en sorte que ledit dispositif (1) puisse s'allonger selon ledit axe longitudinal (L) entrainant la diminution du diamètre au repos (r) du corps tubulaire principal (2) sous l'application d'une tension longitudinale exercée manuellement par le praticien,
ledit procédé comprend une première étape de fabrication d'au moins une structure tricotée de forme tubulaire d'axe longitudinal (L) ayant des première et seconde extrémités libres ouvertes ainsi que des faces extérieure (2c) et intérieure (2d) opposées, à partir de fil(s) monofilament(s) et/ou de fil(s) multifilamentaire(s), ledit procédé comprend une seconde étape au cours de laquelle lesdites première (3a) et seconde (4a) extrémités libres sont enroulées sur elle-même, de préférence à l'aide d'une tige de mise en forme puis thermofixées, en sorte de former respectivement des premier (3) et second (4) rebords annulaires se projetant de la face extérieure (2c) d'un corps tubulaire principal tricoté, lesdits premier (3) et second (4) rebords ayant une configuration en U retourné en sorte de former des première (5) et seconde zones annulaires d'appui.

12. Procédé de fabrication (1) selon la revendication 11, **caractérisé en ce qu'**au cours de la seconde étape, lesdites première (3a) et seconde (4a) extrémités sont enroulées sur elles-mêmes en sorte que leurs bords libres convergent vers la face extérieure (2c) dudit corps tubulaire principal (2) et forment respectivement des première (7) et seconde (8) branches convergeant vers la face extérieure (2c) dudit corps principal (2), de préférence lesdites première (7) et seconde (8) branches sont dans des plans (P1,P2) sensiblement perpendiculaires à l'axe longitudinale (L) dudit corps principal (2).

13. Procédé de fabrication (1) selon l'une ou l'autre des revendications 11 et 12, **caractérisé en ce que** le schéma de maille de la structure tricotée de forme tubulaire est un jersey avant ou arrière et est déterminé en sorte de ménager des colonnes de mailles (12) distinctes et espacées d'une distance (d) supérieure ou égale à 2 mm, de préférence supérieure ou égale à 5 mm.

14. Procédé de fabrication (1) selon l'une quelconque des revendications 11 à 13, d'un dispositif anastomotique selon l'une quelconque des revendications 2 à 7.

## Patentansprüche

1. Anastomosevorrichtung (1) zum Verbinden von leeren Lumen oder Eingeweiden, die geeignet ist, sich an der Oberfläche von zwei benachbarten übereinandergelegten Lumen auszubreiten, wobei die Vorrichtung (1) einen rohrförmigen Hauptkörper (2) mit einer Längsachse (L) umfasst, der ein erstes (2a) und zweites offenes Ende (2b) sowie eine gegenüberliegende Außen- (2c) und Innenfläche (2d) aufweist, wobei das erste (2a) und zweite Ende (2b) durch einen ersten (3) und zweiten Ringbund (4) verlängert sind, die von der Außenfläche (2c) des rohrförmigen Hauptkörpers (2) hervorstehen und eine umgedrehte U-Konfiguration aufweisen, um einen ersten (5) und zweiten ringförmigen Auflagebereich zu bilden, **dadurch gekennzeichnet, dass** der Hauptkörper (2) und der erste (3) und zweite Bund (4) aus einer rohrförmigen gestrickten Struktur, umfassend mindestens zwei Maschenstäbchen (12), mit einer Längsachse (l) gebildet sind, die zu der Achse (L) parallel verläuft, sodass sich die Vorrichtung (1) entlang der Längsachse (L) ausdehnen kann, was im Ruhezustand (r) unter der Anwendung einer Längsspannung, die manuell vom Arzt ausgeübt wird, die Verringerung des Durchmessers des Hauptkörpers (2) mit sich bringt.

2. Anastomosevorrichtung (1) nach Anspruch 1, **dadurch gekennzeichnet, dass** das erste (3a) und zweite freie Ende (4a) des ersten (3) und zweiten Bunds (4) jeweils durch einen ersten (7) und zweiten (8) Arm verlängert sind, die zu der Außenfläche (2c) des rohrförmigen Hauptkörpers (2) verlaufen, wobei sich der erste (7) und zweite Arm (8) vorzugsweise in den Ebenen (P1, P2) befinden, die im Wesentlichen zu der Längsachse (L) des rohrförmigen Hauptkörpers (2) senkrecht verlaufen.

3. Anastomosevorrichtung (1) nach einem der Ansprüche 1 und 2, **dadurch gekennzeichnet, dass** der Hauptkörper (2), der erste (3) und zweite Bund (4), gegebenenfalls der erste (7) und zweite Arm (8), Maschenstäbchen (12) umfassen, die um einen Abstand (d) größer gleich 2 mm, vorzugsweise größer gleich 5 mm, beabstandet sind.

4. Anastomosevorrichtung (1) nach einem der Ansprüche 1 bis 3, **dadurch gekennzeichnet, dass** der Hauptkörper (2), der erste (3) und zweite Bund (4), gegebenenfalls der erste (7) und zweite Arm (8), mindestens sechs Maschenstäbchen (12) umfassen.

5. Anastomosevorrichtung (1) nach einem der Ansprüche 2 bis 4, **dadurch gekennzeichnet, dass** die freien Enden des ersten und zweiten Arms um sich selbst gewickelt sind.

6. Anastomosevorrichtung (1) nach einem der Ansprüche 1 bis 5, **dadurch gekennzeichnet, dass** die mindestens eine rohrförmige gestrickte Struktur einen oder mehrere Monofilamentfaden bzw. Monofilamentfäden und/oder einen oder mehrere Multifilamentfaden bzw. Multifilamentfäden in einer superelastischen Legierung umfasst.

7. Anastomosevorrichtung (1) nach einem der Ansprüche 1 bis 5, **dadurch gekennzeichnet, dass** die mindestens eine rohrförmige gestrickte Struktur einen oder mehrere Monofilamentfaden bzw. Monofilamentfäden und/oder einen oder mehrere Multifilamentfaden bzw. Multifilamentfäden in einer Formgedächtnislegierung umfasst.

8. Implantierbare Vorrichtung (1), umfassend eine Anastomosevorrichtung nach Anspruch 1 und eine weiche, rohrförmige Hülle (20), vorzugsweise aus einem Textilmaterial, die ein distales (20b) und proximales offenes Ende (20a) aufweist, wobei das distale Ende (20b) mit dem ersten Ende des rohrförmigen Hauptkörpers (2) fest verbunden ist oder geeignet ist, damit fest verbunden zu sein.

9. Implantierbare Vorrichtung (1) nach Anspruch 8, **dadurch gekennzeichnet, dass** sie einen Haltering (21) umfasst, der mit dem proximalen Ende (20a) der rohrförmigen Hülle (20) fest verbunden ist oder geeignet ist, damit fest verbunden zu sein, und so ausgestaltet ist, dass er an der Wand der Speiseröhre (22) anliegt.

10. Implantierbare Vorrichtung nach einem der Ansprüche 8 und 9, **dadurch gekennzeichnet, dass** sie eine Anastomosevorrichtung nach einem der Ansprüche 2 bis 7 umfasst.

11. Herstellungsverfahren einer Anastomosevorrichtung (1) nach einem der Ansprüche 1 bis 10 zum Verbinden von leeren Lumen oder Eingeweiden, wobei die Vorrichtung geeignet ist, sich an der Oberfläche von zwei benachbarten übereinandergelegten Lumen auszubreiten, wobei die Vorrichtung (1) einen rohrförmigen Hauptkörper (2) mit einer Längsachse (L) umfasst, der ein erstes (2a) und zweites offenes Ende (2b) sowie eine gegenüberliegende Außen- (2c) und Innenfläche (2d) aufweist, wobei das erste (2a) und zweite Ende (2b) durch einen ersten (3) und zweiten Ringbund (4) verlängert sind, die von der Außenfläche (2c) des rohrförmigen Hauptkörpers (2) hervorstehen und eine umgedrehte U-Konfiguration aufweisen, um einen ersten (5) und zweiten ringförmigen Auflagebereich zu bilden, **dadurch gekennzeichnet, dass** der Hauptkörper (2) und der erste (3) und zweite Bund (4) aus einer rohrförmigen gestrickten Struktur, umfassend mindestens zwei Maschenstäbchen (12), mit einer Längsachse (l) gebildet sind, die zu der Achse (L) parallel verläuft, sodass sich die Vorrichtung (1) entlang der Längsachse (L) ausdehnen kann, was im Ruhezustand (r) unter der Anwendung einer Längsspannung, die manuell vom Arzt ausgeübt wird, die Verringerung des Durchmessers des rohrförmigen Hauptkörpers (2) mit sich bringt,
wobei das Verfahren einen ersten Schritt der Herstellung von mindestens einer rohrförmigen gestrickten Struktur mit einer Längsachse (L), die ein erstes und zweites offenes freies Ende sowie eine gegenüberliegende Außen- (2c) und Innenfläche (2d) aufweist, aus einem Monofilamentfaden bzw. Monofilamentfäden und/oder einem Multifilamentfaden bzw. Multifilamentfäden umfasst, wobei das Verfahren einen zweiten Schritt umfasst, in dessen Verlauf das erste (3a) und zweite freie Ende (4a) um sich selbst gewickelt, vorzugsweise mithilfe eines Formstabs und anschließend thermofixiert werden, um jeweils einen ersten (3) und zweiten Ringbund (4) zu bilden, die von der Außenfläche (2c) eines gestrickten rohrförmigen Hauptkörpers hervorstehen, wobei der erste (3) und zweite Bund (4) eine umgedrehte U-Konfiguration aufweisen, um einen ersten (5) und zweiten ringförmigen Auflagebereich zu bilden.

12. Herstellungsverfahren (1) nach Anspruch 11, **dadurch gekennzeichnet, dass** im Verlauf des zweiten Schritts das erste (3a) und zweite Ende (4a) um sich selbst gewickelt werden, sodass ihre freien Kanten zu der Außenfläche (2c) des rohrförmigen Hauptkörpers (2) verlaufen und jeweils den ersten (7) und zweiten Arm (8) bilden, die zu der Außenfläche (2c) des rohrförmigen Hauptkörpers (2) verlaufen, wobei sich der erste (7) und zweite Arm (8) vorzugsweise in den Ebenen (P1, P2) befinden, die im Wesentlichen zu der Längsachse (L) des Hauptkörpers (2) senkrecht verlaufen.

13. Herstellungsverfahren nach einem der Ansprüche 11 und 12, **dadurch gekennzeichnet, dass** das Maschenschema der rohrförmigen gestrickten Struktur ein vorderes oder hinteres Jersey ist und bestimmt wird, um die Maschenstäbchen (12) zu schonen, die sich um einen Abstand (d) größer gleich 2 mm, vorzugsweise größer gleich 5 mm, unterscheiden und um diesen beabstandet sind.

14. Herstellungsverfahren (1) nach einem der Ansprüche 11 bis 13 einer Anastomosevorrichtung nach einem der Ansprüche 2 bis 7.

## Claims

1. An anastomotic device (1) for joining lumens or hollow viscera, able to be deployed on the surface of two superimposed adjacent lumens, said device (1) comprises a main tubular body (2) with longitudinal axis (L), having open first (2a) and second (2b) ends as well as opposite outer (2c) and inner (2d) faces,
wherein said first (2a) and second (2b) ends extend by first (3) and second (4) annular rims projecting from the outer face (2c) of said main tubular body (2), having an inverted U configuration so as to form first (5) and second annular bearing zones, **characterized in that** said main body (2) and said first (3) and second (4) rims are formed from a tubular knitted structure comprising at least two columns of stitches (12), with longitudinal axis (l) parallel to said axis (L) such that said device (1) can lie along said longitudinal axis (L) causing the decrease of the diameter (r) of the main tubular body (2) at rest under the application of a longitudinal tension exerted manually by the practitioner.

2. The anastomotic device (1) according to claim 1, **characterized in that** the first (3a) and second (4a) free ends of the first (3) and second (4) rims respectively extend by first (7) and second (8) branches converging toward the outer surface (2c) of said main tubular body (2), preferably said first (7) and second (8) branches are in planes (P1, P2) substantially perpendicular to the longitudinal axis (L) of said main tubular body (2).

3. The anastomotic device (1) according to one or the other of claims 1 and 2, **characterized in that** the main body (2), the first (3) and second (4) rims, optionally the first (7) and second (8) branches, comprise columns of stitches (12) spaced apart by a distance (d) greater than or equal to 2 mm, preferably greater than or equal to 5 mm.

4. The anastomotic device (1) according to one of claims 1 to 3, **characterized in that** the main body (2), the first (3) and second (4) rims, optionally the first (7) and second (8) branches, comprise at least six columns of stitches (12).

5. The anastomotic device (1) according to one of claims 2 to 4, **characterized in that** said first and second branches have their free ends wound around themselves.

6. The anastomotic device (1) according to one of claims 1 to 5, **characterized in that** said at least one tubular knitted structure comprises one or several monofilament yarn(s) and/or one or several multi-filament yarn(s) in a superelastic alloy.

7. The anastomotic device (1) according to one or the other of claims 1 to 5, **characterized in that** said at least one tubular knitted structure comprises one or several monofilament yarn(s) and/or one or several multifilament yarn(s) in a shape memory alloy.

8. An implantable device (1) comprising an anastomotic device according to claim 1, and a flexible tubular sheath (20), preferably made from a textile material, having open distal (20b) and proximal (20a) ends, said distal end (20b) being secured or able to be secured to said first end of the main tubular body (2).

9. The implantable device (1) according to claim 8, **characterized in that** it comprises a holding ring (21) secured or able to be secured to said proximal end (20a) of the tubular sheath (20), and configured so as to hold itself bearing against the walls of the esophagus (22).

10. The implantable device according to one or the other of claims 8 and 9, **characterized in that** it comprises an anastomotic device according to any one of claims 2 to 7.

11. A method for manufacturing an anastomotic device (1) according to any one of claims 1 to 10, for joining lumens or hollow viscera, the device being able to be deployed on the surface of two superimposed adjacent lumens, said device (1) comprises a main tubular body (2) with longitudinal axis (L), having open first (2a) and second (2b) ends as well as opposite outer (2c) and inner (2d) faces, said first (2a) and second (2b) ends extending by first (3) and second (4) annular rims projecting from the outer face (2c) of said main tubular body (2), having an inverted U configuration so as to form first (5) and second annular bearing zones, **characterized in that** said main body (2) and said first (3) and second (4) rims are formed from a tubular knitted structure comprising at least two columns of stitches (12), with longitudinal axis (I) parallel to said axis (L) such that said device (1) can lie along said longitudinal axis (L) causing the decrease of the diameter (r) of the main tubular body (2) at rest under the application of a longitudinal tension exerted manually by the practitioner,
said method comprises a first step for manufacturing at least one tubular knitted structure with longitudinal axis (L) having first and second open free ends as well as opposite outer (2c) and inner (2d) faces, from monofilament and/or multifilament yarn(s), said method comprises a second step during which said first (3a) and second (4a) free ends are wound around themselves, preferably using a shaping rod, then heat-set, so as to respectively form first (3) and second (4) annular rims projecting from the outer face (2c) of a knitted main tubular body, said first (3) and second (4) rims having an inverted U configuration so as to form first (5) and second annular bearing zones.

12. The manufacturing method (1) according to claim 11, **characterized in that** during the second step, said first (3a) and second (4a) ends are wound around themselves such that their free ends converge toward the outer face (2c) of said main tubular body (2) and respectively form first (7) and second (8) branches converging toward the outer face (2c) of said main body (2), preferably said first (7) and second (8) branches are in planes (P1, P2) substantially perpendicular to the longitudinal axis (L) of said main body (2).

13. The manufacturing method (1) according to one or the other of claims 11 and 12, **characterized in that** the knitting pattern of the tubular knitted structure is a front or back jersey and is determined so as to arrange separate columns of stitches (12) spaced apart by a distance (d) greater than or equal to 2 mm, preferably greater than or equal to 5 mm.

14. The manufacturing method (1) according to any one of claims 11 to 13, of an anastomotic device according to any one of claims 2 to 7.
